# EUROPEAN PATENT SPECIFICATION

(11) **EP 2 273 981 B1**
(45) Date of publication and mention of the grant of the patent: **05.11.2014**
(21) Application number: 09746710.4
(22) Date of filing: 14.05.2009
(51) Int. Cl.: A61K 9/16, A61K 31/4709

(54) **A SOLID PHARMACEUTICAL FORMULATION**
FESTE PHARMAZEUTISCHE FORMULIERUNG
FORMULATION PHARMACEUTIQUE SOLIDE

(30) Priority: 15.05.2008 JP 2008128259
(43) Date of publication of application: 19.01.2011
(73) Proprietor: Otsuka Pharmaceutical Co., Ltd., Tokyo 101-8535 (JP)
(72) Inventor: MUKAI, Tadashi, Osaka-shi Osaka 541-0045 (JP)
(74) Representative: Von Kreisler Selting Werner - Partnerschaft von Patentanwälten und Rechtsanwälten mbB
(86) International application number: PCT/JP2009/059300
(87) International publication number: WO 2009/139504

(56) References cited:
- EP-A1- 0 397 447
- WO-A1-00/57881
- WO-A1-2004/039361
- WO-A1-2005/113009
- WO-A1-2007/105229
- WO-A1-2008/044862
- WO-A1-2008/054121
- WO-A1-2009/118764
- WO-A2-2009/051022
- US-A- 5 843 479
- US-A1- 2004 197 398
- US-B1- 7 138 143
- DATABASE WPI Week 200275 Thomson Scientific, London, GB; AN 2002-694008 XP002575140 -& JP 2002 193792 A (LION CORP) 10 July 2002 (2002-07-10)
- HEINAMAKI J ET AL: "Formulations releasing the drug proximal to the pylorus in the dog" INTERNATIONAL JOURNAL OF PHARMACEUTICS, ELSEVIER BV, NL LNKD- DOI:10.1016/0378-5173(88)90247-5, vol. 48, no. 1-3, 1 December 1988 (1988-12-01), pages 51-61, XP025531019 ISSN: 0378-5173 [retrieved on 1988-12-01]
- OSTERWALD H P: "Properties of film-formers and their use in aqueous systems" PHARMACEUTICAL RESEARCH, KLUWER ACADEMIC PUBLISHERS, NEW YORK, NY, US, vol. 2, no. 1, 1 January 1985 (1985-01-01) , pages 14-18, XP008123757 ISSN: 0724-8741

## Description

### TECHNICAL FIELD

The present invention relates to a solid pharmaceutical formulation. More particularly, it relates to a sustained-release solid pharmaceutical formulation comprising (a) cilostazol as the active medical ingredient, (b) a pre-gelatinized starch in an amount of 10 to 90 % by weight based on the whole weight of the formulation, (c) one or more kinds of enteric ingredients and (d) an organic acid.

### BACKGROUND ART

A sustained-release solid pharmaceutical formulation is a useful formulation since it can control the blood concentration of an active ingredient to improve the administration condition (e.g. reducing administration frequency), to improve the duration of an active ingredient which has a short half-life in vivo, to reduce a side-effect of an active ingredient whose concentration difference between the minimum blood concentration and the side-effect onset concentration is small, and so on.

Cilostazol is 6-[4-(1-cyclohexyl-1H-tetrazol-5-yl)butoxy]-3,4-dihydrocarbostyril as shown in the following formula (1), which exhibits high inhibitory action for platelet aggregation as well as inhibitory action for phosphodiesterase, antiulcer activity, hypotensive action, antiphlogistic action, etc. and thereby is widely used as an antithrombotic agent, a drug for improving cerebral circulation, an antiinflammatory agent, an antiulcer drug, an antihypertensive drug, an antiasthmatic drug, a phosphodiesterase inhibitor, etc. The cilostazol tablets which are called Pletaal tablet 50^{®} and Pletaal tablet 100^{®} (OTSUKA PHARMACEUTICAL CO., LTD.) have already been on sale (JP-63(1988)-20235-B). In addition, cilostazol is also used for improving various ischemic diseases such as ulcer, pain and coldness that are symptoms based on chronic arterial occlusion because cilostazol also exhibits a peripheral vasodilatation.

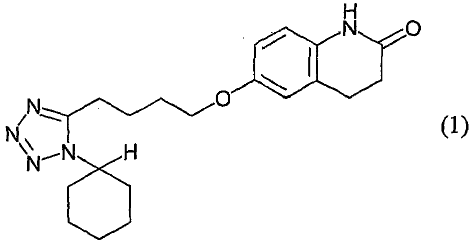

Although the conventional cilostazol tablets are generally used in an oral administration of twice per day for an adult, it has been desirable for elderly patients who are typical patients suffering from the indicated disease to improve the administration frequency, preferably to develop a sustained-release formulation thereof which enables an oral administration of once per day. In addition, it has been also desirable to develop a formulation thereof which is not so affected by a diet because the cilostazol tablet is apt to be affected by a diet, i.e. when administering after meal, Cₘₐₓ increases 2.3 fold and AUC increases 1.4 fold, compared with fasting administration.

In addition, the vasodilatation of cilostazol can lead to a useful effect such as increase of blood flow, but on the contrary, it may cause headache or palpitation. Some patients applied to cilostazol sometimes suffer from headache as a side-effect of cilostazol. In order to reduce the onset of headache, it is necessary to lessen the maximum plasma level of cilostazol and additionally to develop a sustained-release formulation of cilostazol which can keep a sufficiently effective plasma level thereof on the repeated administration.

In general, an oral pharmaceutical formulation has to pass through the stomach which is a strong acid region, and through the intestine which is a neutral to basic region. Therefore, in order to develop a sustained-release formulation of an oral pharmaceutical medicament, it is necessary to consider making a medicament reach to the lower gastrointestinal tract (lower small intestine and large intestine) through various pH-regions. Furthermore, in order to enable a once-daily administration or keep an effective plasma level of a medicament lessening Cₘₐₓ for reducing a side-effect, it is necessary to sufficiently release a medicament from a sustained-release formulation even after the formulation reach the lower gastrointestinal tract.

An enteric formulation coated with an enteric coat is known as a sustained-release formulation which has a sustained-release technique in the lower gastrointestinal tract. However, the technique requires a film coating operation in order to control the drug release via the coat, thus the process of the preparation is troublesome.

On the other hand, a matrix formulation which is formulated for sustained-release using a methacrylic enteric polymer is also known, which is thought to have a possibility of controlling a detailed drug release thanks to the sharp pH-responsiveness of an enteric polymer compound. As an example of such formulation, a matrix formulation which is prepared by compressing a mixture of an enteric polymer compound and a medicament is known (e.g. JP-4(1992)-43049-B, JP- 6(1994)-199657-A, US 4,968,508, US-2006/0159753-A). In case of such tablet formulation, however, its surface area is small and its solubility is low. Therefore, a formulation comprising a hardly soluble medicament whose dissolution rate is late may have some trouble about its drug release.

In addition, JP-6(1994)-24991-A discloses a formulation which is prepared by wet-kneading a powder mixture comprising methacrylic acid copolymer S with ethanol and then by an extrusion granulation technique. This formulation is a granule which is suitably dispersible in gastrointestinal tract, and hence it is possible to lessen the variation between individuals about drug absorption more than the case of a tablet formulation. However, there was a manufacturing problem in case that the amount of enteric polymer compound in the matrix formulation was increased to sufficiently control the drug release.

Although a variety of methods for controlling the drug release were studied as mentioned above, in most of the methods there was a possibility that the drug release rate is changed in the small intestine because of burst phenomenon or intraintestinal pH variation

A starch has been generally used as an excipient for pharmaceutical formulation because of its excellent stability and safety and further when it is used as an additive for a granule prepared by extrusion granulation technique, it gives easier procedure of the granulation step. However, it has a disadvantage lowering the robustness of a solid formulation, and the drug-release property of an administered formulation containing a starch is not so good.

The present inventors had extensively studied a variety of formulations containing a starch as an excipient to solve the above-mentioned problem and had already found that a solid pharmaceutical formulation which is prepared by pre-gelatinizing a starch after mixing a medicament and the starch or after granulating the mixture has a high physical robustness of formulation, an excellent drug-release property of an administered formulation and an excellent digestibility of the excipient (WO 2005/113009). With regard to some medicaments such as cilostazol, however, it has been still necessary to further control the drug-release rate between small intestine and large intestine which are regions located after amylase treatment in order to sustain its absorption.

### DISCLOSURE OF INVENTION

An object of the present invention is to solve the above-mentioned problems involved in the solid pharmaceutical formulation containing cilostazol and the active medical ingredient and a pre-gelatinized starch as an excipient. Namely, an object of the present invention is to provide a sustained-release solid pharmaceutical formulation which can control the drug-release property in the lower gastrointestinal tract which still has not been sufficient, can decrease administration frequency, can reduce a side-effect by lessening Cₘₐₓ, and so on.

The present inventors have extensively studied to reach the above object and then have found that the drug release rate after receiving amylase treatment can be controlled by the technique which an enteric ingredient is added to a pre-gelatinized starch formulation that is a matrix. And also, the present inventors have found that the formulation profile can be further improved by selecting a certain kind of enteric ingredients, and additionally have found that the pharmacokinetics can be affected by adding an organic acid thereto. Based upon the new findings, the present invention has been completed.

The present invention relates to the following inventions.

The present invention provides a solid pharmaceutical formulation comprising (a) cilostazol as the active medical ingredient, (b) a pre-gelatinized starch (α-starch) in an amount of 10 to 90 % by weight based on the whole weight of the formulation, (c) one or more kinds of enteric ingredient(s) and (d) an organic acid. The active medical ingredient mentioned herein refers to the hardly soluble medicament cilostazol. The preferable solid pharmaceutical formulation is a sustained-release solid pharmaceutical formulation.

Also, the present invention provides the above-mentioned solid pharmaceutical formulation unless the enteric ingredient(s) in (c) consist of only methacrylic acid copolymer LD.

In addition, the present invention provides the above-mentioned solid pharmaceutical formulation wherein the one or more kinds of enteric ingredients comprise hydroxypropyl methylcellulose phthalate, hydroxypropyl methylcellulose acetate succinate, carboxymethylethylcellulose, methacrylic acid copolymer L, and/or methacrylic acid copolymer S.

Further, the above-mentioned enteric ingredient(s) prepferably comprise a methacrylic acid copolymer unless the enteric ingredient(s) consist of only methacrylic acid copolymer LD. Preferably, the one or more kinds of enteric ingredients comprise methacrylic acid copolymer L and/or methacrylic acid copolymer S, and more preferably, methacrylic acid copolymer L and methacrylic acid copolymer S. The ratio of methacrylic acid copolymer L and methacrylic acid copolymer S is not limited thereto, but preferably, 30:70 to 70:30, 40:60 to 60:40, or about 50:50.

Furthermore, the present invention provides a solid pharmaceutical formulation which comprises an organic acid. The organic acid is contained in 0.5 to 5 % by weight, preferably 1 to 3 % by weight, more preferably about 2 % by weight, based on the whole weight of the formulation. The preferable organic acid is, for example, citric acid. Furthermore, the combination of methacrylic acid copolymer S as an enteric ingredient and citric acid as an organic acid is preferred.

The present invention provides the above-mentioned solid pharmaceutical formulation wherein the material of the pre-gelatinized starch is one or more starches selected from corn starch, wheat starch, potato starch, rice starch, cassava starch, and tapioca starch. A preferable material of the pre-gelatinized starch is corn starch.

And, the present invention provides the above-mentioned solid pharmaceutical formulation which is in the form of a particle, or in the form of a granule or a powder. The granule or the powder herein is preferably prepared by an extrusion granulation technique. In addition, the present invention provides a capsule formulation which comprises either the particle solid pharmaceutical formulation or the granular/powdery solid pharmaceutical formulation, or both of them; or a tablet formulation which comprises either the particle solid pharmaceutical formulation or the granular/powdery solid pharmaceutical formulation, or both of them.

Further, the present invention provides a method for preparing the above-mentioned solid pharmaceutical formulation which comprises the following steps (i) and (ii): (i) mixing an active medical ingredient, a starch and one or more kinds of enteric ingredients to prepare a starting composition, and (ii) subjecting the starting composition to a pre-gelatinization of starch. The starch in step (i) includes one or a mixture consisting of a starch, a partly pre-gelatinized starch and/or a pre-gelatinized starch, provided that the starch in step (i) is not only a pre-gelatinized starch. The present invention also provides the above-mentioned method for preparing the solid pharmaceutical formulation wherein the starch is pre-gelatinized by heating during the process of the preparation. In addition, the present invention provides a method for preparing the above-mentioned solid pharmaceutical formulation wherein the heating is accompanied with humidification. Furthermore, the present invention provides a solid pharmaceutical formulation which is prepared by the above-mentioned method.

And, the present invention provides a capsule formulation which is filled with a rapidly-release granule or powder containing an active medical ingredient, and the sustained-release granule or powder mentioned above.

The solid pharmaceutical formulation and the method of the preparation thereof are described in detail below.

### Solid pharmaceutical formulation

The solid pharmaceutical formulation of the present invention comprises (a) cilostazol as the active medical ingredient, (b) a pre-gelatinized starch, (c) one or more kinds of enteric ingredients and (d) an organic acid.

The solid pharmaceutical formulation of the present invention contains usually 10 to 90 % by weight, preferably 20 to 80 % by weight, more preferably 25 to 70 % by weight, of the pre-gelatinized starch (a-starch) based on the whole weight of the formulation. By adding the pre-gelatinized starch in such amount to the formulation, the solid pharmaceutical formulation of the present invention has the desired characteristics such as high physical robustness of the formulation and the desired effective and sustained release of the active ingredient within the digestive tract when administered.

The starch raw material to be used herein is not limited to, but includes any conventional starches such as corn starch, wheat starch, potato starch, rice starch, cassava starch, tapioca starch, which may be used in a single kind of the starches or in combination of two or more kinds of the starches. The starch raw material to be used herein may be a mixture with a partly pre-gelatinized starch and/or pre-gelatinized starch of the above-mentioned starch. The pre-gelatinization of the starch may be carried out during the preparation process of the solid formulation, preferably, in the form of a pharmaceutical composition obtained by mixing the starch with the active ingredient and other pharmaceutical carriers. The pre-gelatinization of the starch includes a partial pre-gelatinization of the starch.

Preferred pre-gelatinized starches are a pre-gelatinized corn starch, a pre-gelatinized potato starch, and a pre-gelatinized wheat starch. Particularly preferred pre-gelatinized starch is a pre-gelatinized corn starch, because corn starch has a uniform particle size of 10 to 30 µm and hence is easily processed into the desired formulations and also the pre-gelatinized product thereof is easily handled, and further because corn starch has lower moisture-absorption characteristics in comparison with other starches.

The enteric ingredient herein used may be any kinds of general enteric ingredients, not limited as far as it is not dissolved in the stomach, but dissolved in the intestine. Actually, the enteric ingredient includes, for example, enteric cellulose derivatives such as cellulose acetate phthalate, cellulose acetate trimellitate, cellulose acetate succinate, methylcellulose phthalate, hydroxypropyl methylcellulose acetate succinate, hydroxylpropyl methylcellulose phthalate, hydroxymethyl-ethylcellulose phthalate, hydroxypropylmethyl cellulose acetate maleate, hydroxypropylmethyl cellulose trimellitate and carboxymethylethylcellulose; vinyl derivatives such as polyvinyl butylate phthalate, polyvinyl alcohol acetate phthalate; enteric acrylate copolymers such as copolymer of methacrylic acid and ethyl acrylate (e.g. methacrylic acid-ethyl acrylate) and copolymer of methacrylic acid and methyl methacrylate (e.g. methacrylic acid copolymer L, methacrylic acid copolymer S). The enteric ingredient may be used alone or in combination of two or more kinds of ingredients. Amongst them, preferred examples are hydroxypropyl methylcellulose acetate succinate, hydroxypropyl methylcellulose phthalate, carboxymethylethylcellulose, methacrylic acid copolymer L, methacrylic acid copolymer S, and more preferred ones are hydroxypropyl methylcellulose acetate succinate, methacrylic acid copolymer L, and methacrylic acid copolymer S. When using two or more enteric ingredients, it is preferred that each pH range at which each enteric ingredient can be dissolved is different each other. In this case, the enteric ingredients include hydroxypropyl methylcellulose acetate succinate, hydroxylpropyl methylcellulose phthalate, carboxymethylethylcellulose, methacrylic acid copolymer L, methacrylic acid copolymer S, and methacrylic acid copolymer LD. Further, it is preferred to combine two kinds of enteric ingredients, one can be dissolved at the pH of 6 or more (e.g. methacrylic acid copolymer L), and the other one can be dissolved at the pH of 7 or more (e.g. methacrylic acid copolymer S). It is also preferred to combine two kinds of enteric ingredients, one can be dissolved at the pH of 5 or more, and the other one can be dissolved at the pH of 6 or more. More preferably, the enteric ingredient is methacrylic acid copolymer L and/or methacrylic acid copolymer S, and even more preferably, methacrylic acid copolymer L and methacrylic acid copolymer S. In addition, when using one kind of enteric ingredient, it is preferred to combine an organic acid with the enteric ingredient. In this case, the preferable enteric ingredient is hydroxypropyl methylcellulose acetate succinate, methacrylic acid copolymer L, or methacrylic acid copolymer S, and the preferable organic acid is citric acid. The enteric ingredient (s) may be incorporated in an amount of 1 to 30 % by weight, preferably 5 to 20 % by weight, more preferably 5 to 15 % by weight, based on the whole weight of the formulation.

The active medical ingredient to be incorporated into the solid pharmaceutical formulation of the present invention is cilostazol.

The active medical ingredient may be used alone or in combination with two or more kinds of medicament. The additional medicaments may be any kinds of medicaments having any pharmaceutical activities as far as they can be administered orally, and may be selected from the following lists.

They may be water-soluble medicaments or hardly water-soluble medicaments. The medicament herein used includes, for example, a conventional medicament, i.e. various medicaments as incorporated in pharmaceutical preparations such as agents for respiratory organs, agents for digestive organs, cardiovascular agents, agents for central nervous system, agents for peripheral nervous system, antibiotics, chemotherapeutics, antitumor agents, platelet aggregation inhibitors, anti-allergic agents, vitamins, or nutrients. Preferred medicaments are hardly water-soluble medicaments. Further preferred medicaments are classified into Class II (High Permeability, Law Solubility) in "waiver of in Vivo Bioavailability and Bioequivalents Studies for Immediate Release Solids Dosage Forms Containing Certain Active Moieties/Active Ingredients Based on Biopharmaceutics Classification System (FDA Guidance)" (in this description, it may occasionally be referred to as "Biopharmaceutics Classification System"). When the hardly water-soluble medicament is formulated into a sustained release preparation, it can release the active medicament gradually and effectively within the digestive organs, and hence can exhibit the desired pharmaceutical activities when administered.

The active ingredient used in the solid pharmaceutical formulation includes, for example, teophylline, grepafloxacin, carteolol, procaterol, rebamipide, aripiprazole, cilostazol, Physuline, tolvaptan, acetaminophen, nifedipine, ketoprophen, naproxen, diclofenac, itraconazole, piroxicam, phenytoin, and verapamil. Amongst them, preferred example is ketoprophen, naproxen, diclofenac, itraconazole, piroxicam, phenytoin, or verapamil.

These active ingredients are particularly useful when they are formulated in a sustained release preparation.

The active ingredient may be incorporated into the solid pharmaceutical formulation in an appropriate amount, which may vary depending on the kind and efficacy of the active ingredient, sexes and ages of the patients to be treated, and so on, but may be contained, for example, in the range of about 0.01 to 70 % by weight, preferably 0.1 to 60 % by weight, more preferably 1 to 60 % by weight, based on the whole weight (in dry weight) of the composition.

The organic acid used in the solid pharmaceutical formulation of the present invention includes, for example, tartaric acid, malic acid, succinic acid, citric acid, maleic acid, acetic acid, oxalic acid, etc. The organic acid may be used alone or in combination of two or more kinds of the organic acids. Amongst them, citric acid is preferable.

The solid pharmaceutical formulation may further comprise an appropriate amount of various other additives such as excipients, binders, pH adjustors, disintegrators, absorption promoters, lubricants, colorants, flavors, perfumes, and the like, unless they give any adverse affect.

These additives are, for example, excipients (e.g. lactose, white sugar, mannitol, sodium chloride, glucose, calcium carbonate, kaolin, crystalline cellulose, silicates); binders (e.g. water, ethanol, simple syrup, aqueous glucose solution, aqueous starch solution, aqueous gelatine solution, carboxymethylcellulose, carboxymethylcellulose sodium, sellac, methyl cellulose, hydroxypropyl methylcellulose, hydroxypropyl cellulose, polyvinylpyrrolidone, polyvinyl alcohol, gelatin, dextrin, pullulan); pH adjustors (e.g. citric acid, citric anhydride, sodium citrate, sodium citrate dihydrate, anhydrous disodium hydrogenphosphate, anhydrous sodium dihydrogenphosphate, sodium hydrogenphosphate, sodium dihydrogen phosphate); disintegrators (e.g. carmellose calcium, low substituted hydroxypropylcellulose, carmellose, crosscarmellose sodium, carboxymethyl starch sodium, crosspovidone); plasticizers (e.g. polysorbate 80); absorption promoters (e.g. quaternary ammonium bases, sodium laurylsulfate); lubricants (e.g. purified talc, stearates, polyethylene glycol, colloidal silicates, sucrose fatty acid esters); colorants (e.g. yellow iron oxide, yellow iron sesquioxide, sesquioxide, ß-carotene, titanium oxide, food colors such as food blue No. 1, copper chlorophyll, riboflavin); flavors (e.g. ascorbic acid, aspartame, sweet hydrangea, sodium chloride, fructose, saccharine, powdered sugar).

The solid pharmaceutical formulation of the present invention may be formulated in any type of solid formulations such as fine powders, particles, powders, granules, tablets, or the like. The solid pharmaceutical formulation of the present invention may further be formulated in the form of coated products or capsules. Amongst these formulations, preferred ones are granules or powders.

In order to form in granules, it is preferable to incorporate crystalline cellulose into the solid pharmaceutical formulation. By adding crystalline cellulose thereto, the composition can easily be formed in spherical shapes by conventional technique for regulating spherical shape, and hence it can be easily granulated by extrusion granulation technique. Besides, the composition is formed into spherical shape, it is easy to package into capsules. It is also advantageous that the spherical composition can be effectively coated. The crystalline cellulose may be incorporated in an amount of 2 to 90 % by weight, preferably 5 to 80 % by weight, more preferably 5 to 70 % by weight, based on the whole weight of the pharmaceutical formulation.

In addition, the capsule formulation of the present invention can keep the blood concentration of an active ingredient constant by filling a rapidly-release granule or powder and a sustained-release granule or a powder which each contain an active medical ingredient.

### Method of preparation

The solid pharmaceutical formulation of the present invention can be prepared by subjecting a composition comprising an active medical ingredient, a starch and an enteric ingredient (hereinafter, referred to "starting compositions") to a pre-gelatinization. In more detail, the solid pharmaceutical formulation can be prepared by the following steps (i) and (ii):
(i) a step of mixing an active medical ingredient, a starch and an enteric ingredient to prepare a starting composition, and
(ii) a step of subjecting the starting composition to a pre-gelatinization of starch.

The components of the starting composition prepared in the step (i) are still maintained in the final solid pharmaceutical formulation, except that the starch is pre-gelatinized to be converted into a-starch in the step (ii). Accordingly, in the step (i), the active medical ingredient, the starch to be pre-gelatinized, and other optional ingredients (various carriers) should be incorporated into the starting composition in the same amounts as the final solid pharmaceutical formulation.

The starting composition contains preferably water in addition to the above-mentioned components. The water content in the starting composition is not limited to specific range, but is usually 30 to 80 % by weight, preferably 40 to 80 % by weight, more preferably 40 to 70 % by weight, based on the whole weight of the starting composition. By adding water in such range as mentioned above, the starting composition can easily be formed into the desired forms and further can be effectively subjected to the subsequent pre-gelatinization step.

The starting composition may be any type of solid formulations, such as fine powders, powders, granules, tablets, and so on. The shape of the starting composition can be maintained even after being subjected to the pre-gelatinization in the step (ii) to form the desired solid pharmaceutical formulation containing the pre-gelatinized starch (a-starch). Accordingly, the starting composition is preferably formed in the same formulation as the final product suitable for the desired drug.

The method for formulation of the starting composition into the desired forms is not limited but it may be prepared by a conventional method. When the solid pharmaceutical formulation is in the form of the granules, it is preferable to incorporate crystalline cellulose into the starting composition (the amount of crystalline cellulose is defined above) and to granulate the composition by extrusion granulation technique in the step (i).

The starting composition thus obtained is subjected to the next step (ii), wherein the starch is pre-gelatinized so as to be converted into a-starch. The pre-gelatinization may be carried out by a conventional method for converting a starch raw material into a-starch. For example, when the starting composition contains water as mentioned above, the starting composition is subjected to heat treatment. The heat treatment may be carried out by any conventional heating treatment, for example, heating with steam, dry heating with hot air, high frequency induction heating, heating with microwave, and the like. The heating temperature may vary according to the heating means, but is usually in the range of 75 to 100°C, preferably 80 to 100°C. The heating time may appropriately be determined according to the heating means by a person skilled in the art.

Besides, when the starting composition does not contain water, the starting composition is preferably subjected to heating with steam, for example, by spraying water onto the starting composition and then treating with a steam microwave, by which the starch is converted into a-starch. The heating conditions are the same as mentioned above.

When the starting composition contains crystalline cellulose and is in the form of granules prepared by the extrusion granulation technique, the granules have uniform spherical shape (high degree of spherical shape), and hence are suitably subjected to microwave heating. When the starting composition having spherical shape of not flat (concavo-convex) surface is subjected to microwave heating, the parts having convex surface are first heated and the moisture is distilled off from the surface, and thereby the pre-gelatinization is done with less efficiency. On the other hand, when the starting composition having spherical shape in high degree is subjected to microwave heating, the starting composition is heated first from inner part and hence the surface keeps the moisture with less evaporation during the heating treatment, and thereby, the pre-gelatinization can effectively be done.

Thus, the starting composition is subjected to the pre-gelatinization of the starch contained in the composition to give the desired formulation containing pre-gelatinized starch (a-starch). The pharmaceutical formulation resulting from the pre-gelatinization contains moisture and hence is preferably subjected to drying in order to remove the moisture. The drying is carried out by a conventional method, for example, by keeping in a drying room at a temperature of 50 to 90°C, preferably 60 to 80°C. The drying time may be optionally determined depending on the type of the pharmaceutical formulation and the drying temperature, and so on by a person skilled in the art.

The solid pharmaceutical formulation of the present invention may be a preparation obtained by the above steps (i) and (ii) and optionally drying step (i.e. the preparation obtained by the pre-gelatinization); or alternatively it may be a preparation obtained by further subjecting "the solid formulation obtained by the pre-gelatinization" to a processing step by any conventional methods which are usually used for preparing pharmaceutical formulations.

For example, the granules obtained by pre-gelatinization may be subjected to tableting to give a solid pharmaceutical formulation in the form of tablets. Besides, the solid pharmaceutical formulation or the further processed product may be subjected to coating to give a coated product. Further, the solid pharmaceutical formulation or the further processed product may be packed into capsules to give the desired solid pharmaceutical formulation in the form of capsules.

The solid pharmaceutical formulation of the present invention can be easily prepared since the enteric ingredient is contained as a matrix, not as a film-coating material. Therefore, the preparation of a granule formulation and a similar formulation of the present invention can be carried out in a shorter time compared with a conventional film-coated formulation and thus it is possible to reduce the manufacturing cost.

In addition, the solid pharmaceutical formulation of the present invention has a high physical robustness of formulation, an excellent drug-release property of an administered formulation, and an excellent digestibility of the excipient, and especially, the drug-release property between small intestine and large intestine is excellent. And, the solid pharmaceutical formulation of the present invention has a property controlling the drug release rate by the action of a certain kind of enteric ingredient and an organic acid after the formulation is digested and disintegrated with amylase in the small intestine. Thereby, it enables a variety of sustained-release properties such as an oral administration of once per day and a reduction of a side-effect.

### BRIEF DESCRIPTION OF DRAWING

Fig. 1 is a graph showing dissolution characteristics of the solid pharmaceutical formulations (in Example 1 and Reference Example 1).
Fig. 2 is a graph showing dissolution characteristics of the solid pharmaceutical formulations (in Example 2 and Reference Example 2).
Fig. 3 is a graph showing dissolution characteristics of the solid pharmaceutical formulations (in Example 3 and Reference Example 3).
Fig. 4 is a graph showing dissolution characteristics of the solid pharmaceutical formulations (in Example 4 and Reference Example 4).
Fig. 5 is a graph showing dissolution characteristics of the solid pharmaceutical formulations (in Example 5 and Reference Example 5).
Fig. 6 is a graph showing dissolution characteristics of the solid pharmaceutical formulations (in Example 6 and Reference Example 6).
Fig. 7 is a graph showing change of average plasma concentration of cilostazol with time on administering the solid pharmaceutical formulations (in Examples 4 - 6 and Reference Example 7)(n = 7, mean ± standard deviation).

### BEST MODE FOR CARRYING OUT THE INVENTION

The present invention is illustrated in more detail by the following examples and experiments. Examples not covered by the scope of the claims are for illustrative purpose.

### Example 1

Corn starch (tradename: "Nisshoku Corn Starch", manufactured by Nippon Shokuhin Kako K.K.)(90 g), crystalline cellulose (tradename: "CEOLUS PH301", manufactured by Asahi Kasei Corporation) (30 g), hydroxypropyl methylcellulose acetate succinate (tradename: "AS-HF", manufactured by Shin-Etsu Chemical Co., Ltd)(30 g), and cilostazol (manufactured by Otsuka Pharmaceutical Co., Ltd.) (150 g) were mixed, and the mixture was put into a speed kneader (Model number: NSK-150, manufactured by Okada Seiko K.K.), then thereto 37.5 mL of 4 % aqueous polysorbate 80 solution (containing 1.5 g of polysorbate 80) and purified water (92.5 g) were added with stirring to give a starting composition (Starting Composition Example 1).

The starting composition was subjected to extrusion granulation with an extrusion granulator equipped with a dome die (hole diameter: 0.6 mm, DomeGran DG-L1, manufactured by DALTON Co. LTD.) to give wet granules. The wet granules were treated with a spheroidizer (Murmerizer QJ-400, manufactured by DALTON Co. LTD.) to regulate the shape and size of the granules, and thereby obtaining the starting composition of wet granules.

The starting composition of wet granules was subjected to heating and humidifying with a steam oven (HEALSIO, manufactured by SHARP CORPORATION) at "warm mode" for 20 minutes so that the corn starch could be pre-gelatinized. The granules thus heated/humidified were dried in an air-drying oven at 60°C for 6 hours to give a solid pharmaceutical formulation in the form of granules (Example 1) .

### Example 2

Corn starch (tradename: "Nisshoku Corn Stanch", manufactured by Nippon Shokuhin Kako K.K.)(90 g), crystalline cellulose (tradename: "CEOLUS PH301", manufactured by Asahi Kasei Corporation) (30 g), hydroxypropyl methylcellulose acetate succinate (tradename: "AS-HF", manufactured by Shin-Etsu Chemical Co., Ltd)(30 g), and cilostazol (manufactured by Otsuka Pharmaceutical Co., Ltd.) (150 g) were mixed, and the mixture was put into a speed kneader (Model number: NSK-150, manufactured by Okada Seiko K.K.), then thereto 37.5 mL of 4 % aqueous polysorbate 80 solution (containing 1.5 g of polysorbate 80) and purified water (67.5 g) were added with stirring to give a starting composition (Starting Composition Example 2).

The starting composition was subjected to extrusion granulation with an extrusion granulator equipped with a dome die (hole diameter: 0.6 mm, DomeGran DG-L1, manufactured by DALTON Co. LTD.) to give wet granules. The wet granules were treated with a spheroidizer (Murmerizer QJ-400, manufactured by DALTON Co. LTD.) to regulate the shape and size of the granules, and thereby obtaining the starting composition of wet granules.

The starting composition of wet granules was subjected to heating and humidifying with a steam oven (HEALSIO, manufactured by SHARP CORPORATION) at "warm mode" for 20 minutes so that the corn starch could be pre-gelatinized. The granules thus heated/humidified were dried in an air-drying oven at 60°C for 6 hours to give a solid pharmaceutical formulation in the form of granules (Example 2).

### Example 3

Corn starch (tradename: "Nisshoku Corn Starch", manufactured by Nippon Shokuhin Kako K.K.)(69 g), crystalline cellulose (tradename: "CEOLUS PH301", manufactured by Asahi Kasei Corporation) (51 g), methacrylic acid copolymer L (tradename: "Eudragit L100", manufactured by EVONIK) (30 g), and cilostazol (manufactured by Otsuka Pharmaceutical Co., Ltd.)(150 g) were mixed, and the mixture was put into a speed kneader (Model number: NSK-150, manufactured by Okada Seiko K.K.), then thereto purified water (130 g) were added with stirring to give a starting composition (Starting Composition Example 3).

The starting composition was subjected to extrusion granulation with an extrusion granulator equipped with a dome die (hole diameter: 0.6 mm, DomeGran DG-L1, manufactured by DALTON Co. LTD.) to give wet granules. The wet granules were treated with a spheroidizer (Murmerizer QJ-400, manufactured by DALTON Co. LTD.) to regulate the shape and size of the granules, and thereby obtaining the starting composition of wet granules.

The starting composition of wet granules was subjected to heating and humidifying with a steam oven (HEALSIO, manufactured by SHARP CORPORATION) at "warm mode" for 20 minutes so that the corn starch could be pre-gelatinized. The granules thus heated/humidified were dried in an air-drying oven at 60°C for 6 hours to give a solid pharmaceutical formulation in the form of granules (Example 3).

### Example 4

Corn starch (tradename: "Nisshoku Corn Starch", manufactured by Nippon Shokuhin Kako K.K.)(69 g), crystalline cellulose (tradename: "CEOLUS PH301", manufactured by Asahi Kasei Corporation) (21 g), methacrylic acid copolymer L (tradename: "Eudragit L100", manufactured by EVONIK) (15 g), methacrylic acid copolymer S (tradename: " Eudragit S100", manufactured by EVONIK) (15 g), a pre-gelatinized starch (tradename: "Amycol C", manufactured by NIPPON STARCH CHEMICAL CO., LTD.) (30 g), and cilostazol (manufactured by Otsuka Pharmaceutical Co., Ltd.)(150 g) were mixed, and the mixture was put into a speed kneader (Model number: NSK-150, manufactured by Okada Seiko K.K.), then thereto 75 mL of 4 % aqueous polysorbate 80 solution (containing 3 g of polysorbate 80) and purified water (20 g) were added with stirring to give a starting composition (Starting Composition Example 4).

The starting composition was subjected to extrusion granulation with an extrusion granulator equipped with a dome die (hole diameter: 0.6 mm, DomeGran DG-L1, manufactured by DALTON Co. LTD.) to give wet granules. The wet granules were treated with a spheroidizer (Murmerizer QJ-400, manufactured by DALTON Co. LTD.) to regulate the shape and size of the granules, and thereby obtaining the starting composition of wet granules.

The starting composition of wet granules was subjected to heating and humidifying with a steam oven (HEALSIO, manufactured by SHARP CORPORATION) at "warm mode" for 20 minutes so that the corn starch could be pre-gelatinized. The granules thus heated/humidified were dried in an air-drying oven at 60°C for 6 hours to give a solid pharmaceutical formulation in the form of granules (Example 4).

### Example 5

Corn starch (tradename: "Nisshoku Corn Starch", manufactured by Nippon Shokuhin Kako K.K.)(69 g), crystalline cellulose (tradename: "CEOLUS PH301", manufactured by Asahi Kasei Corporation) (21 g), methacrylic acid copolymer S (tradename: " Eudragit S100", manufactured by EVONIK) (30 g), a pre-gelatinized starch (tradename: "LYCATAB PGS", manufactured by ROQUETTE)(30 g), and cilostazol (manufactured by Otsuka Pharmaceutical Co., Ltd.) (150 g) were mixed, and the mixture was put into a speed kneader (Model number: NSK-150, manufactured by Okada Seiko K.K.), then thereto 75 mL of 4 % aqueous polysorbate 80 solution (containing 3 g of polysorbate 80) and purified water (20 g) were added with stirring to give a starting composition (Starting Composition Example 5).

The starting composition was subjected to extrusion granulation with an extrusion granulator equipped with a dome die (hole diameter: 0.6 mm, DomeGran DG-L1, manufactured by DALTON Co. LTD.) to give wet granules. The wet granules were treated with a spheroidizer (Murmerizer QJ-400, manufactured by DALTON Co. LTD.) to regulate the shape and size of the granules, and thereby obtaining the starting composition of wet granules.

The starting composition of wet granules was subjected to heating and humidifying with a steam oven (HEALSIO, manufactured by SHARP CORPORATION) at "warm mode" for 20 minutes so that the corn starch could be pre-gelatinized. The granules thus heated/humidified were dried in an air-drying oven at 60°C for 6 hours to give a solid pharmaceutical formulation in the form of granules (Example 5).

### Example 6

Corn starch (tradename: "Nisshoku Corn Starch", manufactured by Nippon Shokuhin Kako K.K.)(69 g), crystalline cellulose (tradename: "CEOLUS pH301", manufactured by Asahi Kasei Corporation) (21 g), methacrylic acid copolymer S (tradename: "Eudragit S100", manufactured by EVONIK) (30 g), a pre-gelatinized starch (tradename: "LYCATAB PGS", manufactured by ROQUETTE)(30 g), citric acid (6 g), and cilostazol (manufactured by Otsuka Pharmaceutical Co., Ltd.)(150 g) were mixed, and the mixture was put into a speed kneader (Model number: NSK-150, manufactured by Okada Seiko K.K.), then thereto 75 mL of 4 % aqueous polysorbate 80 solution (containing 3 g of polysorbate 80) and purified water (20 g) were added with stirring to give a starting composition (Starting Composition Example 6).

The starting composition was subjected to extrusion granulation with an extrusion granulator equipped with a dome die (hole diameter: 0.5 mm, DomeGran DG-L1, manufactured by DALTON Co. LTD.) to give wet granules. The wet granules were treated with a spheroidizer (Murmerizer QJ-400, manufactured by DALTON Co. LTD.) to regulate the shape and size of the granules, and thereby obtaining the starting composition of wet granules.

The starting composition of wet granules was subjected to heating and humidifying with a steam oven (HEALSIO, manufactured by SHARP CORPORATION) at "warm mode" for 20 minutes so that the corn starch could be pre-gelatinized. The granules thus heated/humidified were dried in an air-drying oven at 60°C for 6 hours to give a solid pharmaceutical formulation in the form of granules (Example 6).

### Reference Examples 1 to 6

Without heating/humidifying treatment, the starting compositions of wet granules (prepared in the procedure in Examples 1 to 6) were directly dried in a air-drying oven at 60°C for 6 hours to give solid pharmaceutical formulations in the form of granules (Reference Examples 1 - 6).

### Experiment 1

Using the solid pharmaceutical formulations of Example 1 and Reference Example 1 which are in the form of granules, the following experiment was carried out. Each of the formulations was weighed out in an amount equivalent to 100 mg of cilostazol, which was subjected to the dissolution test according to the following condition: Test solution: 900 mL of 4 % aqueous polysorbate 80 solution; Paddle method (100 rpm). The UV absorption of the samples collected in each predetermined time was measured to determine the dissolution rate of the medicament. Two hours after starting the test, 0.18 g of amylase (tradename: "a-Amylase", manufactured by Wako Pure Chemical Industries, Limited) was added to each test solution, and 4 hours after starting the test, a buffer solution was added thereto to adjust pH of each test solution to 7.0.

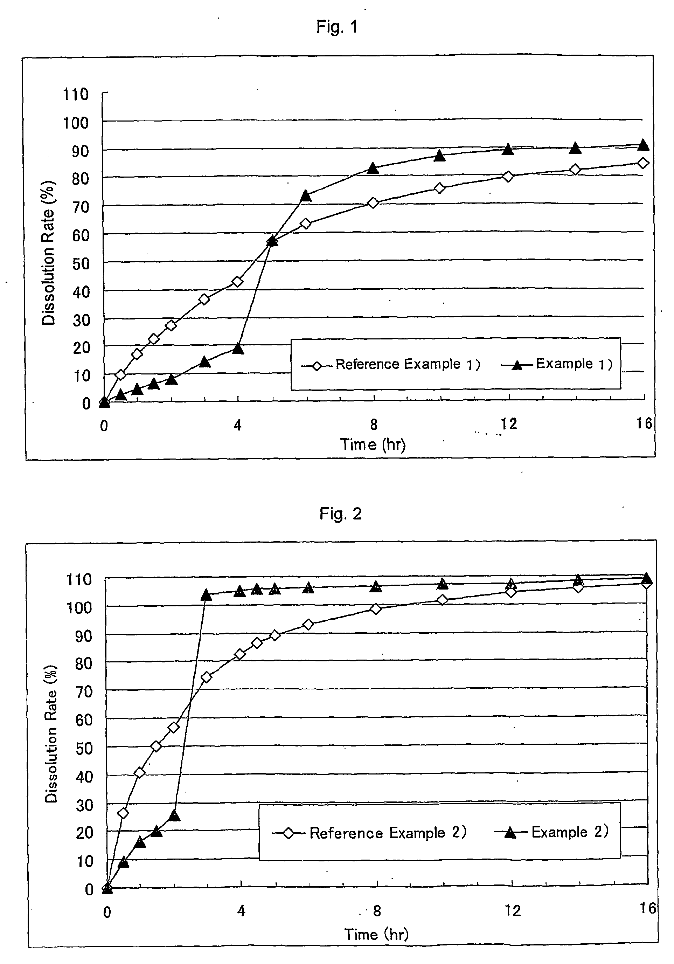

The results are shown in Fig. 1. With regard to Reference Example 1, the dissolution thereof began immediately after starting the dissolution test, and the drug release rate was hardly affected with an addition of amylase or upper-change of pH. While, with regard to Example 1, the drug release rate sharply rose 4 hours after the dissolution test started, i.e. at the time when pH was changed; and after 6 hours, the drug release rate of Example 1 exceeded the rate of Reference Example 1.

### Experiment 2

Using the solid pharmaceutical formulations of Example 2 and Reference Example 2 which are in the form of granules, the following experiment was carried out. Each of the formulations was weighed out in an amount equivalent to 100 mg of cilostazol, which was subjected to the dissolution test according to the following condition: Test solution: 900 mL of 0.3 % aqueous sodium laurylsulfate solution; Paddle method (100 rpm). The UV absorption of the samples collected in each predetermined time was measured to determine the dissolution rate of the medicament. Two hours after starting the test, 0.18 g of amylase (tradename: "a-Amylase", manufactured by Wako Pure Chemical Industries, Limited) was added to each test solution, and 4 hours after starting the test, a buffer solution was added thereto to adjust pH of each test solution to 7.1.

The results are shown in Fig. 2. 2 hours after starting the test, by the addition of amylase, the medicament of Example 2 was rapidly dissolved, and thereby it was confirmed that the action of the pre-gelatinized starch had been kept.

### Experiments 3 - 6

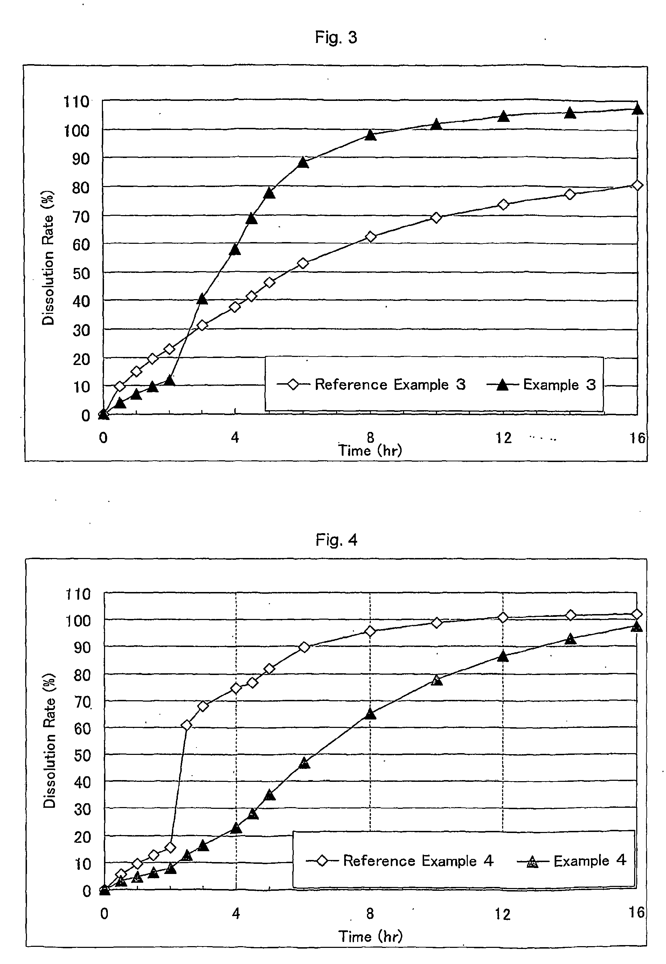

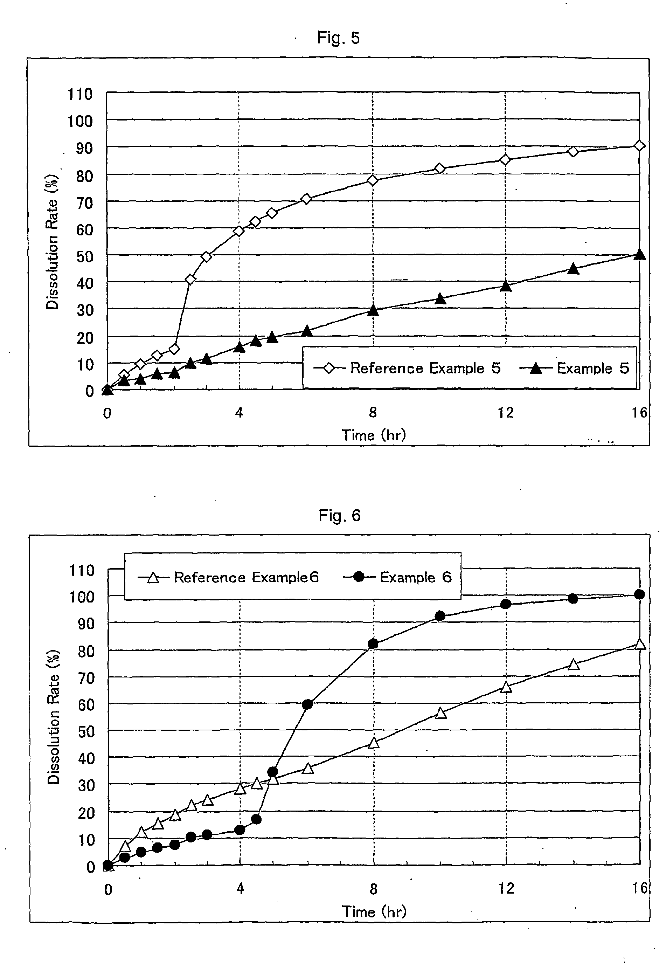

Using Examples 3 - 6 and Reference Examples 3 - 6, the same test as Experiment 2 was carried out (Experiments 3 - 6). However, the pH resetting 4 hours after starting the dissolution test was "7.1" for Experiments 3, 4 and 5, and "7.5" for Experiment 6. In all cases, as the result of Experiment 2, the dissolutions of Examples 3 - 6 were more sustained than those of Reference Examples 3 - 6 (Fig. 3 - 6).

In conclusion, according to the results of Experiments 1 - 6, it has been found that the formulations of the present invention have two properties, i.e. releasing a medicament in each formulation containing pre-gelatinized starch due to the action that amylase digests the starch, and additionally controlling the drug release depending on pH due to the addition of an enteric ingredient.

### Evaluation of Pharmacokinetics

Using the granules containing cilostazol prepared in Examples 4 - 6, each granule which contains 100 mg of cilostazol was administered to 7 healthy adult men after meal, and then the blood collection was carried out with time and the concentration of cilostazol in blood was measured.

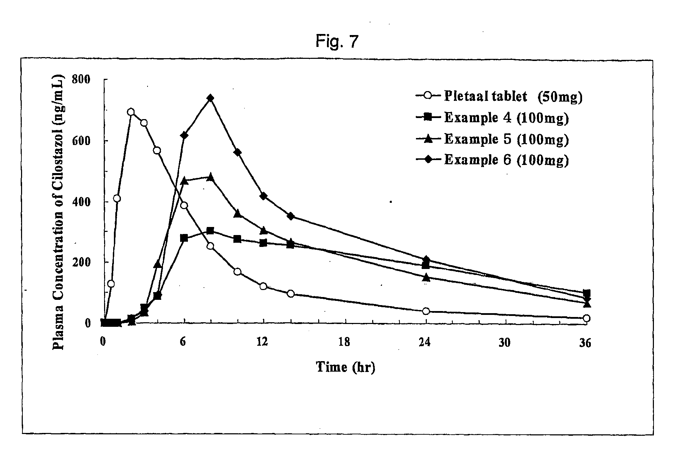

And, in the same manner, commercially available Pletaal tablet^{®} (containing 50 mg of cilostazol)(Reference Example 7) was administered to 7 healthy adult men after meal, and then the blood collection was carried out with time and the concentration of cilostazol in blood was measured. The results are shown in Table 1 and Fig.7.

Compared with Reference Example, the time-point of Cₘₐₓ in all Examples could be delayed. Amongst these results, the result in Example 6 which contained the organic acid exhibited higher Cₘₐₓ and AUC than those of Examples 4 and 5. While, the formulations of Examples 4 and 5 (especially, Example 4) exhibited a lessened Cₘₐₓ compared with Reference Example, and additionally exhibited a pharmacokinetic profile which can keep an activity of a medicament for a long term.

Methacrylic acid copolymer L is an enteric ingredient which can be dissolved at pH 6 or higher, and methacrylic acid copolymer S is an enteric ingredient which can be dissolved at pH 7 or higher. According to the results of Experiments 3 - 5 wherein the dissolution rates of Examples 3 - 5 are shown respectively, it has been found that the higher the pH range at which the contained enteric ingredient can be dissolved is, the slower the dissolution rate is in order to delay the dissolution.

As shown in Fig. 7, however, when each granule of the present invention containing cilostazol was actually administered to healthy adults, the result was inconsistent with the result of dissolution tests (see, the results of Example 4 and Example 5). Namely, the granule whose pH range at which the enteric ingredient can be dissolved is lower (i.e. Example 4, which has lower pH-range thereof than Example 5) has a profile to make the dissolution gradual and make the efficacy sustainedly-maintained.

And, it has been found that the solid pharmaceutical formulation containing a combination of two or more enteric ingredients which have different pH-ranges for dissolution (like Example 4) has a pharmacokinetic profile to lessen the Cₘₐₓ and keep the blood level constant to make the efficacy sustainedly-maintained for a long period after the administration, compared with the cases using one kind of enteric ingredient.

On the other hand, in the case of Example 6 which contains an enteric ingredient having a higher pH-range for dissolution and further contains an organic acid, it was thought that the dissolution is delayed more and more even if the pH around the medicament rises since the organic acid delays the actual pH-rising. However, in both the result of the dissolution test in Experiment 6 and the result of the pharmacokinetic test herein, the medicament in Example 6 was sharply dissolved after running up the pH range at which the enteric ingredient can be dissolved, though a little delaying of dissolution was observed. It has been found that the solid pharmaceutical formulation further containing an organic acid (e.g. citric acid) together with an enteric ingredient (like Example 6) has a pharmacokinetic profile to dissolve a sufficient amount of medicament for a short period after pH is changed, while lowering the initial dissolution.

Based on the findings, it has become possible to design a sustained-release formulation to fit any purpose by suitably selecting out the compositions of the present invention, and also it might be possible to design a sustained-release formulation which can keep a constant blood level of a medicament for a long term by combining the present sustained-release formulation and a normal non-sustained-release formulation.

**[Table 1]**

| Formulation | AUCₜ (ng·hr/mL) | Cₘₐₓ (ng/mL) | Tₘₐₓ (hr) | AUC_{∞} (ng·hr/mL) |
|---|---|---|---|---|
| Reference Example 7 (50 mg) | 5555 | 715 | 2.4 | 5973 |
| Example 4 (100 mg) | 6679 | 351 | 11.1 | 15114 |
| Example 5 (100 mg) | 7282 | 524 | 7.1 | 12029 |
| Example 6 (100 mg) | 9810 | 747 | 7.4 | 11305 |

| | | | | |
|---|---|---|---|---|
| Note) "hr" indicates hour(s). AUCₜ: Area Under a Curve of correlation between the plasma level and elapse of time (in trapezoidal rule) AUC_{∞}: Area Under a Curve of correlation between the plasma level and elapse of time till infinite time Cₘₐₓ: Maximum plasma concentration Tₘₐₓ: Time-to- maximum plasma concentration | | | | |

## Claims

1. A solid pharmaceutical formulation comprising
(a) cilostazol, (b) a pre-gelatinized starch in an amount of 10 to 90 % by weight based on the whole weight of the formulation, (c) one or more kinds of enteric ingredients and (d) an organic acid.

2. The solid pharmaceutical formulation of claim 1 wherein the organic acid is contained in 0.5 to 5 % by weight.

3. The solid pharmaceutical formulation of claim 1 or 2 wherein the organic acid is citric acid.

4. The solid pharmaceutical formulation of any one of claims 1 to 3 wherein the one or more kinds of enteric ingredients comprise hydroxypropyl methylcellulose acetate succinate, hydroxypropyl methylcellulose phthalate, carboxymethylethylcellulose, methacrylic acid copolymer L and/or methacrylic acid copolymer S.

5. The solid pharmaceutical formulation of claim 3 wherein the enteric ingredients comprise methacrylic acid copolymer S.

6. The solid pharmaceutical formulation of any one of claims 1 to 5 wherein the pre-gelatinized starch is pre-gelatinized corn starch.

7. The solid pharmaceutical formulation of any one of claims 1 to 6 which is prepared by the following steps (i) and (ii):
(i) mixing cilostazol, a starch, one or more kinds of enteric ingredients and an organic acid to prepare a starting composition, and
(ii) subjecting the starting composition to a pre-gelatinization of starch.

8. The solid pharmaceutical formulation of claim 7 wherein the starch in (i) is one or a mixture consisting of a starch, a partly pre-gelatinized starch and/or a pre-gelatinized starch, provided that the starch in (i) is not only a pre-gelatinized starch.

9. The solid pharmaceutical formulation of any one of claims 1 to 8 which is in the form of a particle.

10. The solid pharmaceutical formulation of any one of claims 1 to 8 which is in the form of a granule or a powder.

11. The solid pharmaceutical formulation of claim 10 which is prepared by an extrusion granulation technique.

12. A capsule formulation which comprises the solid pharmaceutical formulation of claims 9, 10 and/or 11.

13. A tablet formulation which comprises the solid pharmaceutical formulation of claims 9, 10 and/or 11.

14. A method for preparing the solid pharmaceutical formulation of any one of claims 1 to 11, wherein the starch is pre-gelatinized by heating during the process of the preparation.

15. The method of claim 14 wherein the heating is accompanied with humidification.

16. The solid pharmaceutical formulation of any one of claims 1 to 11 which is prepared by the method of claim 14 or 15.

17. A capsule formulation which is filled with a rapidly-release granule or powder containing cilostazol, and the granule or powder of any one or more of claims 9 to 11.

## Patentansprüche

1. Feste pharmazeutische Zubereitung, umfassend:
(a) Cilostazol, (b) eine vorgelatinisierte Stärke in einer Menge von 10 bis 90 Gew.-%, bezogen auf das Gesamtgewicht der Zubereitung, (c) eine oder mehrere Arten von magensaftresistenten Bestandteilen und (d) eine organische Säure.

2. Feste pharmazeutische Zubereitung gemäß Anspruch 1, wobei die organische Säure in einem Anteil von 0,5 bis 5 Gew.-% enthalten ist.

3. Feste pharmazeutische Zubereitung gemäß Anspruch 1 oder 2, wobei es sich bei der organischen Säure um Zitronensäure handelt.

4. Feste pharmazeutische Zubereitung gemäß einem der Ansprüche 1 bis 3, wobei die eine oder die mehreren Arten von magensaftresistenten Bestandteilen Hydroxypropylmethylcelluloseacetatsuccinat, Hydroxypropylmethylcellulosephthalat, Carboxymethylethylcellulose, Methacrylsäure-Copolymer L und/oder Methacrylsäure-Copolymer S umfassen.

5. Feste pharmazeutische Zubereitung gemäß Anspruch 3, wobei die magensaftresistenten Bestandteile Methacrylsäure-Copolymer S umfassen.

6. Feste pharmazeutische Zubereitung gemäß einem der Ansprüche 1 bis 5, wobei die vorgelatinisierte Stärke vorgelatinisierte Maisstärke ist.

7. Feste pharmazeutische Zubereitung gemäß einem der Ansprüche 1 bis 6, die durch die folgenden Schritte (i) und (ii) hergestellt wird:
(i) Mischen von Cilostazol, einer Stärke, einer oder mehreren Arten von magensaftresistenten Bestandteilen und einer organischen Säure, um eine Ausgangszusammensetzung herzustellen; und
(ii) Vorgelatinisieren der Stärke in der Ausgangszusammensetzung.

8. Feste pharmazeutische Zubereitung gemäß Anspruch 7, wobei es sich bei der Stärke in (i) um eine oder ein Gemisch, das aus einer Stärke, einer zum Teil vorgelatinisierten Stärke und/oder einer vorgelatinisierten Stärke besteht, handelt, mit der Maßgabe, dass die Stärke in (i) nicht nur eine vorgelatinisierte Stärke ist.

9. Feste pharmazeutische Zubereitung gemäß einem der Ansprüche 1 bis 8, die in Form von Teilchen vorliegt.

10. Feste pharmazeutische Zubereitung gemäß einem der Ansprüche 1 bis 8, die in Form eines Granulats oder Pulvers vorliegt.

11. Feste pharmazeutische Zubereitung gemäß Anspruch 10, die durch eine Extrusionsgranulierungstechnik hergestellt wird.

12. Kapselzubereitung, die die feste pharmazeutische Zubereitung gemäß den Ansprüchen 9, 10 und/oder 11 umfasst.

13. Tablettenzubereitung, die die feste pharmazeutische Zubereitung gemäß den Ansprüchen 9, 10 und/oder 11 umfasst.

14. Verfahren zur Herstellung der festen pharmazeutischen Zubereitung gemäß einem der Ansprüche 1 bis 11, wobei die Stärke durch Erhitzen während des Vorgangs der Herstellung vorgelatinisiert wird.

15. Verfahren gemäß Anspruch 14, wobei das Erhitzen mit einer Befeuchtung einhergeht.

16. Feste pharmazeutische Zubereitung gemäß einem der Ansprüche 1 bis 11, die nach dem Verfahren gemäß Anspruch 14 oder 15 hergestellt ist.

17. Kapselzubereitung, die mit einem Granulat oder Pulver mit schneller Wirkstofffreisetzung, das Cilostazol enthält, und dem Granulat oder Pulver gemäß einem oder mehreren der Ansprüche 9 bis 11 gefüllt ist.

## Revendications

1. Formulation pharmaceutique solide comprenant (a) du cilostazol, (b) un amidon prégélatinisé en une quantité de 10 à 90 % en poids par rapport au poids total de la formulation, (c) un ou plusieurs types d'ingrédients entérosolubles, et (d) un acide organique.

2. Formulation pharmaceutique solide selon la revendication 1, dans laquelle l'acide organique est présent en une quantité de 0,5 à 5 % en poids.

3. Formulation pharmaceutique solide selon la revendication 1 ou 2, dans laquelle l'acide organique est l'acide citrique.

4. Formulation pharmaceutique solide selon l'une quelconque des revendications 1 à 3, dans laquelle le ou les types d'ingrédients entérosolubles comprennent l'acétosuccinate d'hydroxypropylméthylcellulose, le phtalate d'hydroxypropylméthylcellulose, la carboxyméthyléthylcellulose, le copolymère L de l'acide méthacrylique et/ou le copolymère S de l'acide méthacrylique.

5. Formulation pharmaceutique solide selon la revendication 3, dans laquelle les ingrédients entérosolubles comprennent le copolymère S de l'acide méthacrylique.

6. Formulation pharmaceutique solide selon l'une quelconque des revendications 1 à 5, dans laquelle l'amidon prégélatinisé est l'amidon de maïs prégélatinisé.

7. Formulation pharmaceutique solide selon l'une quelconque des revendications 1 à 6, qui est préparée par les étapes (i) et (ii) ci-après :
(i) mélange de cilostazol, d'un amidon, d'un ou plusieurs types d'ingrédients entérosolubles et d'un acide organique pour préparer une composition de départ, et
(ii) soumission de la composition de départ à une prégélatinisation de l'amidon.

8. Formulation pharmaceutique solide selon la revendication 7, dans laquelle l'amidon de (i) consiste en :'un amidon, un amidon partiellement prégélatinisé et/ou un amidon prégélatinisé, ou un de leurs mélanges, à la condition que l'amidon de (i) ne soit pas seulement un amidon prégélatinisé.

9. Formulation pharmaceutique solide selon l'une quelconque des revendications 1 à 8, qui se présente sous la forme d'une particule.

10. Formulation pharmaceutique solide selon l'une quelconque des revendications 1 à 8, qui se présente sous la forme d'un granule ou d'une poudre.

11. Formulation pharmaceutique solide selon la revendication 10, qui est préparée par une technique de granulation par extrusion.

12. Formulation de gélule, qui comprend la formulation pharmaceutique solide selon les revendications 9, 10 et/ou 11.

13. Formulation de comprimé qui comprend la formulation pharmaceutique solide selon les revendications 9, 10 et/ou 11.

14. Procédé de préparation de la formulation pharmaceutique solide selon l'une quelconque des revendications 1 à 11, dans lequel l'amidon est prégélatinisé par chauffage pendant le processus de préparation.

15. Procédé selon la revendication 14, dans lequel le chauffage est accompagné d'une humidification.

16. Formulation pharmaceutique solide selon l'une quelconque des revendications 1 à 11, qui est préparée par le procédé de la revendication 14 ou 15.

17. Formulation de gélule qui est remplie d'un granule ou d'une poudre à libération rapide contenant du cilostazol, et le granule ou la poudre selon l'une quelconque, ou plus, des revendications 9 à 11.
